# EUROPEAN PATENT SPECIFICATION

(11) **EP 3 238 604 B1**
(45) Date of publication and mention of the grant of the patent: **08.12.2021**
(21) Application number: 15872976.4
(22) Date of filing: 21.12.2015
(51) Int. Cl.: A61B 1/00

(54) **PROCESSOR DEVICE AND CONTROL PROGRAM FOR AN ENDOSCOPE**
PROZESSORVORRICHTUNG UND STEUERUNGSPROGRAMM FÜR EIN ENDOSKOP
DISPOSITIF DE PROCESSEUR ET PROGRAMME DE COMMANDE POUR UN ENDOSCOPE

(30) Priority: 22.12.2014 JP 2014258795
(43) Date of publication of application: 01.11.2017
(73) Proprietor: FUJIFILM Corporation, Tokyo 106-8620 (JP)
(72) Inventor: KAKU, Toshihiko, Kanagawa 258-8538 (JP)
(74) Representative: Jeffrey, Philip Michael
(86) International application number: PCT/JP2015/085644
(87) International publication number: WO 2016/104408

(56) References cited:
- JP-A- 2002 065 581
- JP-A- 2006 061 683
- JP-A- 2007 061 638
- JP-A- 2011 139 734
- JP-A- 2011 217 798
- JP-A- 2013 005 830
- JP-A- 2013 255 656
- JP-A- 2014 188 083
- JP-A- 2014 226 341
- JP-B2- 2 918 162
- JP-B2- 2 918 162
- US-A1- 2011 245 642
- US-A1- 2013 041 218

## Description

### Field of the Invention

The present invention relates to a processor device for an endoscope for calculating blood vessel index values, such as a blood vessel density, a blood vessel thickness, and a blood vessel length, an operation method thereof, and a non-transitory computer readable medium.

### Description of the Related Art

In the medical field, diagnosis using an endoscope system including a light source device, an endoscope, and a processor device has been widely performed. The light source device generates illumination light used for illumination of an observation target. The endoscope images the observation target illuminated with the illumination light using an imaging sensor, and outputs an image signal. The processor device generates an image of the observation target from the image signal output from the endoscope, and displays the image on a monitor.

In the endoscope field, in addition to normal observation for observing the observation target illuminated with visible light, such as white light, for example, fluorescence observation for observing fluorescence emitted from the observation target or biological information observation for checking the state of the observation object using biological information, such as oxygen saturation, a blood vessel thickness, a blood vessel length, and a blood vessel density, as disclosed in JP5461753B has been performed. US 2013/041218 is concerned with an endoscopic device that irradiates a plurality of illuminating light having different spectrums from each other onto a subject. JP 2014188083 is concerned with an endoscope system for calculating a degree of oxygen saturation.

### SUMMARY OF THE INVENTION

### Problems to Be Solved by the Invention

Among the pieces of biological information used for biological information observation, a blood vessel index value obtained by digitizing information regarding a blood vessel, such as the thickness or the length of a blood vessel, changes with an imaging range change according to an observation distance change. In the case of diagnosis using the blood vessel index value that changes with the observation distance as described above, there are several problems. For example, in a case where diagnostic criteria according to a blood vessel index value is defined at a specific observation distance (for example, an observation distance in magnified observation), it is necessary to acquire an image in accordance with the specific observation distance and calculate the blood vessel index value of the specific observation distance from the acquired image.

However, it may be difficult to accurately adjust the observation distance to a specific observation distance. In addition, in a case where the quality of an image acquired at a specific observation distance is not good even assuming that the observation distance is accurately adjusted to the specific observation distance, it is difficult to accurately calculate the blood vessel index value of the specific observation distance in many cases.

Although a method of correcting fluorescence according to the observation distance is disclosed in JP5461753B, the correction method is to adjust the fluorescence intensity according to the observation distance, such as increasing the fluorescence intensity in a distant view and decreasing the fluorescence intensity in a near view. Therefore, even assuming that the correction method disclosed in JP5461753B is used, it is not possible to acquire the blood vessel index value at a specific observation distance.

It is an object of the present invention to provide a processor device for an endoscope capable of acquiring a blood vessel index value of a specific observation distance used for predetermined diagnostic criteria, an operation method thereof, and a non-transitory computer readable medium.

### Means for Solving the Problems

In order to achieve the aforementioned object, a processor device for an endoscope is provided as claimed in claim 1.

A non-transitory computer readable medium is provided as claimed in claim 9.

It is preferable that the distance-invariant blood vessel index value is the density.

It is preferable to further comprise a storage unit that stores the first blood vessel index value of the first observation distance. It is preferable that the storage unit further stores the distance ratio.

It is preferable to further comprise a unit conversion unit that converts the second blood vessel index value of the specific observation distance from a number of pixels to a specific unit.

It is preferable that the distance acquisition unit further acquires a second observation distance as the specific observation distance, the distance ratio calculation unit calculates, as a first distance ratio, the distance ratio in a case where the specific observation distance is set to the second observation distance, and the index value correction unit acquires a blood vessel index value of the second observation distance as the second blood vessel index value of the specific observation distance by correcting the distance variant blood vessel index values of the first blood vessel index value of the first observation distance according to the first distance ratio. It is preferable that the endoscope further comprise: an imaging optical system capable of changing an imaging magnification between the first observation distance and the second observation distance; and an imaging magnification change operation unit for inputting an imaging magnification change instruction to change the imaging magnification to the imaging optical system. It is preferable that the distance acquisition unit acquires the first observation distance and the second observation distance based on the imaging magnification change instruction.

It is preferable to further comprise a reference observation distance setting unit that sets a predetermined reference observation distance. It is preferable that the distance ratio calculation unit calculates, as a second distance ratio, the distance ratio in a case where the specific observation distance is set to the reference observation distance and that the index value correction unit acquires a blood vessel index value of the reference observation distance as the second blood vessel index value of the specific observation distance by correcting the distance variant blood vessel index values of the first blood vessel index value of the first observation distance according to the second distance ratio.

### Effect of the Invention

According to the present invention, since the blood vessel index value of the specific observation distance is acquired by correcting the blood vessel index value of the first observation distance according to the distance ratio calculated from the first observation distance and the specific observation distance, it is possible to provide a processor device for an endoscope capable of acquiring the blood vessel index value of the specific observation distance used for predetermined diagnostic criteria, and a control program.

### BRIEF DESCRIPTION OF THE DRAWINGS

Fig. 1 is an external view of an endoscope system.
Fig. 2 is a block diagram showing a function of the endoscope system.
Fig. 3 is a graph showing the spectra of violet light, blue light, green light, and red light.
Fig. 4 is a graph showing the spectral characteristics of a color filter.
Fig. 5 is a block diagram showing a function of a measurement image processing unit.
Figs. 6A and 6B are explanatory diagrams illustrating a non-magnified observation distance and a magnified observation distance.
Fig. 7 is a schematic diagram showing an image signal and a blood vessel index value of the non-magnified observation distance.
Fig. 8 is a block diagram showing a function of an index value correction section.
Fig. 9 is a schematic diagram showing a blood vessel index value of the non-magnified observation distance and a blood vessel index value of the magnified observation distance.
Fig. 10 is a schematic diagram of a pseudo-color special image signal.
Fig. 11 is a schematic diagram of a monitor on which information indicating a blood vessel index value is displayed.
Fig. 12 is a flowchart of a first embodiment.
Fig. 13 is a block diagram showing a storage unit.
Fig. 14 is a block diagram showing a unit conversion section.
Fig. 15 is a block diagram showing a reference observation distance setting section.
Fig. 16 is a block diagram showing a function of an endoscope system of a second embodiment.
Fig. 17 is a graph showing the spectrum of white light.
Fig. 18 is a graph showing the spectrum of special light.
Fig. 19 is a block diagram showing a function of an endoscope system of a second embodiment.
Fig. 20 is a plan view showing a rotary filter.

### DESCRIPTION OF THE PREFERRED EMBODIMENTS

### [First embodiment]

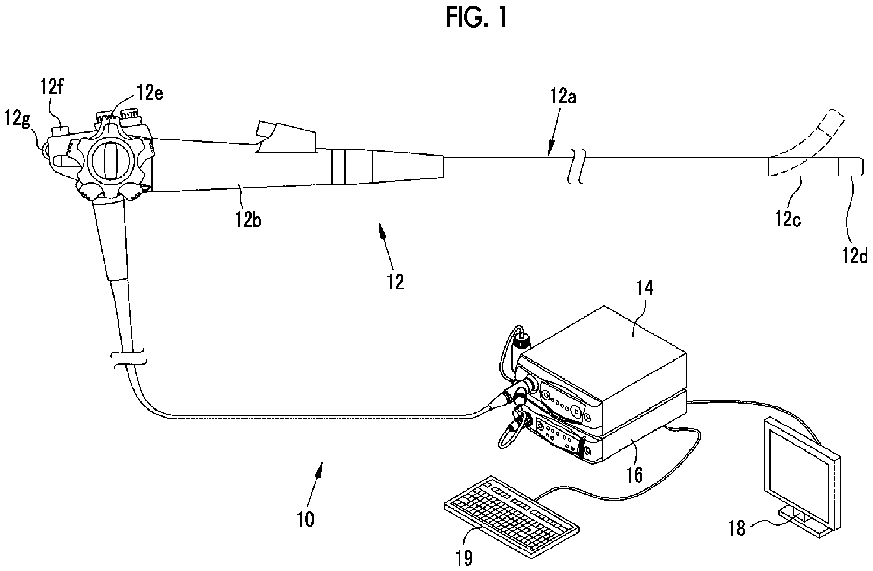

As shown in Fig. 1, an endoscope system 10 includes an endoscope 12, a light source device 14, a processor device 16, a monitor 18, and a console 19. The endoscope 12 is optically connected to the light source device 14. The endoscope 12 is electrically connected to the processor device 16. The endoscope 12 includes an insertion unit 12a that is inserted into the body of an observation target, an operation unit 12b provided in the proximal end portion of the insertion unit 12a, and a bending portion 12c and a distal end portion 12d that are provided at the distal end side of the insertion unit 12a. In the event that a doctor operates an angle knob 12e of the operation unit 12b, the bending portion 12c is bent. The bending operation makes it possible to direct the distal end portion 12d in a desired direction.

In addition to the angle knob 12e, a mode selector switch 12f and a zoom operation unit 12g as an imaging magnification change operation unit are provided in the operation unit 12b. The mode selector switch 12f is used for an observation mode switching operation. The endoscope system 10 has two observation modes of a normal mode and a measurement mode. In the normal mode, an image of natural colors obtained by imaging an observation target using white light as illumination light (hereinafter, referred to as a normal image) is displayed on the monitor 18. In the measurement mode, a blood vessel index value of a blood vessel of the observation target is measured, and an image based on the blood vessel index value (hereinafter, referred to as a special image) is displayed on the monitor 18. The zoom operation unit 12g is used to input an imaging magnification change instruction for instructing an imaging optical system 30b, which will be described later, to change the imaging magnification between magnified observation in which the observation target is displayed in an enlarged manner and non-magnified observation in which magnified observation is not performed.

The processor device 16 is electrically connected to the monitor 18 and the console 19. The monitor 18 displays an image of the observation target, information attached to the image of the observation target, and the like. The console 19 functions as a user interface for receiving an input operation, such as function setting. In addition, an external recording medium (not shown) in which an image, image information, and the like are recorded may be connected to the processor device 16.

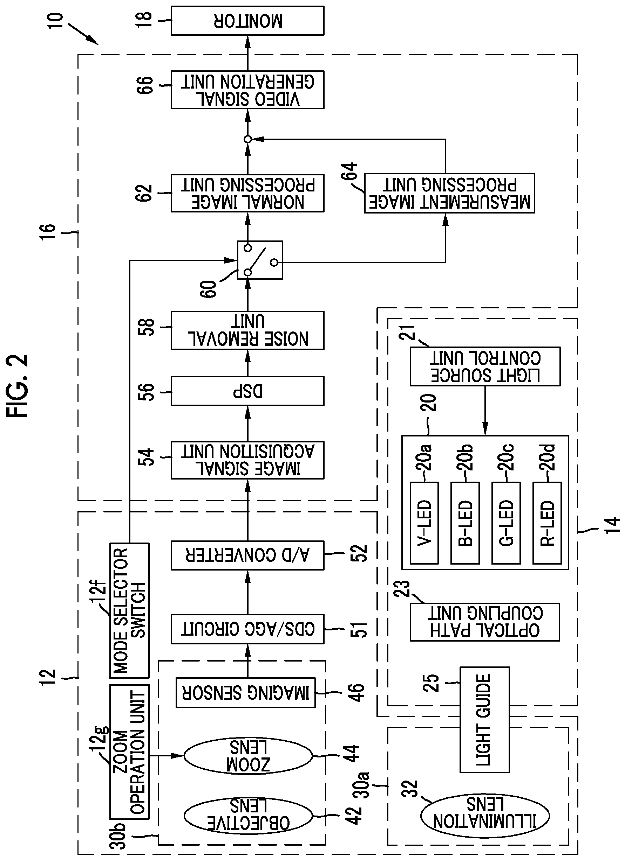

As shown in Fig. 2, the light source device 14 includes a light source 20 and a light source control unit 21 that controls the light source 20. The light source 20 has a plurality of semiconductor light sources, for example. The light source 20 turns on or off each semiconductor light source, and emits illumination light by controlling the amount of light emitted from each semiconductor light source in the case of turning on each semiconductor light source. In the present embodiment, the light source 20 includes LEDs of four colors of a violet light emitting diode (V-LED) 20a, a blue light emitting diode (B-LED) 20b, a green light emitting diode (G-LED) 20c, and a red light emitting diode (R-LED) 20d.

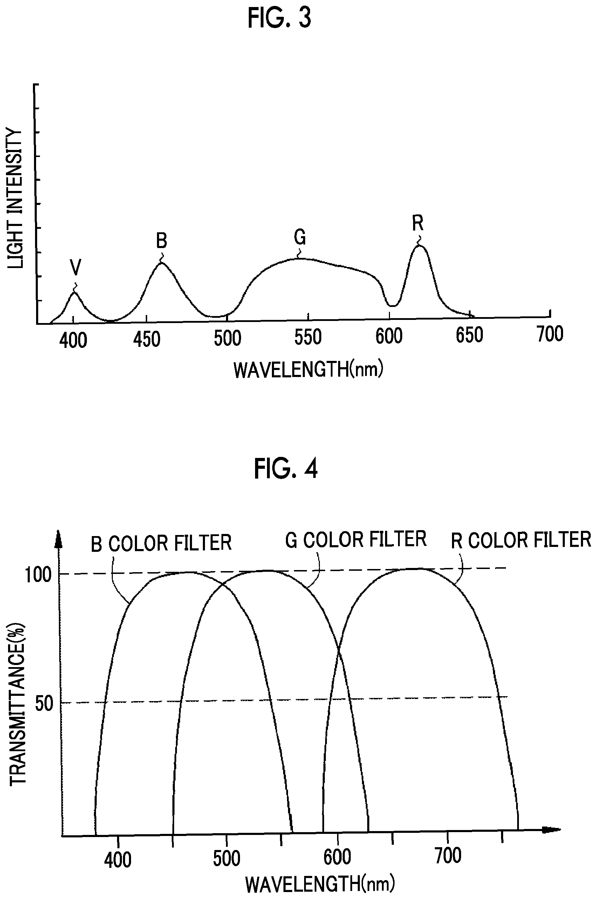

As shown in Fig. 3, the V-LED 20a is a violet semiconductor light source that emits violet light V in a wavelength band of 380 nm to 420 nm that has a center wavelength of 405 nm. The B-LED 20b is a blue semiconductor light source that emits blue light B in a wavelength band of 420 nm to 500 nm that has a center wavelength of 460 nm. The G-LED 20c is a green semiconductor light source that emits green light G in a wavelength band of 480 nm to 600 nm. The R-LED 20d is a red semiconductor light source that emits red light R in a wavelength band of 600 nm to 650 nm that has a center wavelength of 620 nm to 630 nm. In addition, the width of the center wavelength of each of the V-LED 20a and the B-LED 20b is about +5 nm to +10 nm.

The light source control unit 21 independently controls ON or OFF of each of the LEDs 20a to 20d, the amount of light emission at the time of lighting, and the like by inputting a control signal to each of the LEDs 20a to 20d. In the present embodiment, in both observation modes of the normal mode and the measurement mode, the light source control unit 21 turns on all of the V-LED 20a, the B-LED 20b, the G-LED 20c, and the R-LED 20d. Therefore, white light including the violet light V, the blue light B, the green light G, and the red light R is used as illumination light in the normal mode and the measurement mode.

Light of each color emitted from each of the LEDs 20a to 20d is incident on a light guide 25, which is inserted into the insertion unit 12a, through an optical path coupling unit 23 formed of a mirror, a lens, or the like. The light guide 25 is built into the endoscope 12 and the universal cord (cord connecting the endoscope 12 with the light source device 14 and the processor device 16). The light guide 25 causes illumination light emitted from the light source 20 to propagate to the distal end portion 12d of the endoscope 12.

An illumination optical system 30a and the imaging optical system 30b are provided in the distal end portion 12d of the endoscope 12. The illumination optical system 30a has an illumination lens 32. The illumination light propagated by the light guide 25 is emitted to the observation target through the illumination lens 32. The imaging optical system 30b includes an objective lens 42, a zoom lens 44, and an imaging sensor 46. Various kinds of light, such as reflected light, scattered light, and fluorescence from the observation target due to emission of illumination light, are incident on the imaging sensor 46 through the objective lens 42 and the zoom lens 44. As a result, an image of the observation target is formed on the imaging sensor 46.

The zoom lens 44 freely moves between the tele end and the wide end according to the imaging magnification change instruction from the zoom operation unit 12g, thereby making it possible to change the imaging magnification. In the event that the zoom lens 44 moves to the wide end, the image of the observation target is magnified. On the other hand, in the event that the zoom lens 44 moves to the tele end, the image of the observation target is reduced. In the event that non-magnified observation is performed, the zoom lens 44 is disposed at the wide end. In the event that magnified observation is performed by doctor's operation using the zoom operation unit 12g, the zoom lens 44 moves from the wide end to the tele end.

The imaging sensor 46 is a color imaging sensor that images an observation target illuminated with illumination light. As shown in Fig. 4, one of color filters of primary colors of B (blue), G (green), and R (red) is provided in each pixel of the imaging sensor 46. Accordingly, the imaging sensor 46 receives violet light to blue light in a B pixel (blue pixel) in which a B color filter is provided, receives green light in a G pixel (blue pixel) in which a G color filter is provided, and receives red light in an R pixel (red pixel) in which an R color filter is provided. Then, the image signal of each color of BGR is output from the pixel of each color of BGR.

As the imaging sensor 46, it is possible to use a charge coupled device (CCD) imaging sensor or a complementary metal oxide semiconductor (CMOS) imaging sensor. Instead of the primary color imaging sensor 46, a complementary color imaging sensor including complementary color filters of cyan (C), magenta (M), yellow (Y), and green (G) may be used. In the case of using the complementary color imaging sensor, image signals of four colors of CMYG are output. Therefore, by converting the image signals of four colors of CMYG into image signals of three colors of RGB by complementary color-primary color conversion, it is possible to obtain the same image signals of RGB colors as in the imaging sensor 46. Instead of the imaging sensor 46, a monochrome imaging sensor in which no color filter is provided may be used.

A correlated double sampling/automatic gain control (CDS/AGC) circuit 51 performs correlated double sampling (CDS) or automatic gain control (AGC) for the analog image signal obtained from the imaging sensor 46. The image signal having passed through the CDS/AGC circuit 51 is converted into a digital image signal by an analog/digital (A/D) converter 52. The digital image signal after A/D conversion is input to the processor device 16.

The processor device 16 includes an image signal acquisition unit 54, a digital signal processor (DSP) 56, a noise removal unit 58, an image processing switching unit 60, a normal image processing unit 62, a measurement image processing unit 64, and a video signal generation unit 66. In addition, a control unit (not shown) as a computer for controlling the operation of each unit in the processor device 16 is provided in the processor device 16. The control unit has a read only memory (ROM) in which a program is stored and a random access memory (RAM) for temporarily storing data. The control unit reads a program from the ROM to the RAM, and controls each unit based on the read program.

The image signal acquisition unit 54 acquires a digital image signal from the imaging sensor 46 through the CDS/AGC circuit 51 and the A/D converter 52.

The DSP 56 performs various kinds of signal processing, such as defect correction processing, offset processing, gain correction processing, linear matrix processing, gamma conversion processing, and demosaic processing, on the acquired image signals. The defect correction processing is for correcting the signal of a defective pixel of the imaging sensor 46. The offset processing is for setting an accurate zero level by removing a dark current component from the image signal after the defect correction processing. The gain correction processing is for adjusting the signal level by multiplying the image signal after the offset processing by a specific gain.

The linear matrix processing improves the color reproducibility of the image signal after the gain correction processing. The gamma conversion processing is for adjusting the brightness or saturation of the image signal after the linear matrix processing. Demosaic processing (also referred to as isotropic processing or synchronization processing) is performed on the image signal after the gamma conversion processing, and the signal of missing color in each pixel is generated by interpolation. Through the demosaic processing, all pixels have signals of RGB colors. The noise removal unit 58 removes noise by performing noise removal processing on the image signal subjected to various kinds of signal processing by the DSP 56. As the noise removal processing, for example, a moving average method, a median filter method, or the like can be used. The image signal after noise has been removed by the noise removal unit 58 is transmitted to the image processing switching unit 60.

The image processing switching unit 60 transmits the image signal to the normal image processing unit 62 in a case where the observation mode is set to the normal mode. On the other hand, the image processing switching unit 60 transmits the image signal to the measurement image processing unit 64 in a case where the observation mode is set to the measurement mode.

The normal image processing unit 62 generates a normal image by performing color conversion processing, color enhancement processing, and structure enhancement processing on the image signal received from the image processing switching unit 60. In the color conversion processing, 3 x 3 matrix processing, gradation conversion processing, three-dimensional look-up table (LUT) processing, and the like are performed on the image signal. The color enhancement processing is performed on the image signal after the color conversion processing. The structure enhancement processing is, for example, processing for enhancing the structure of the observation target, such as a blood vessel or a pit pattern (gland duct), and is performed on the image signal after the color enhancement processing. A color image using the image signal subjected to various kinds of image processing and the like as described above is a normal image.

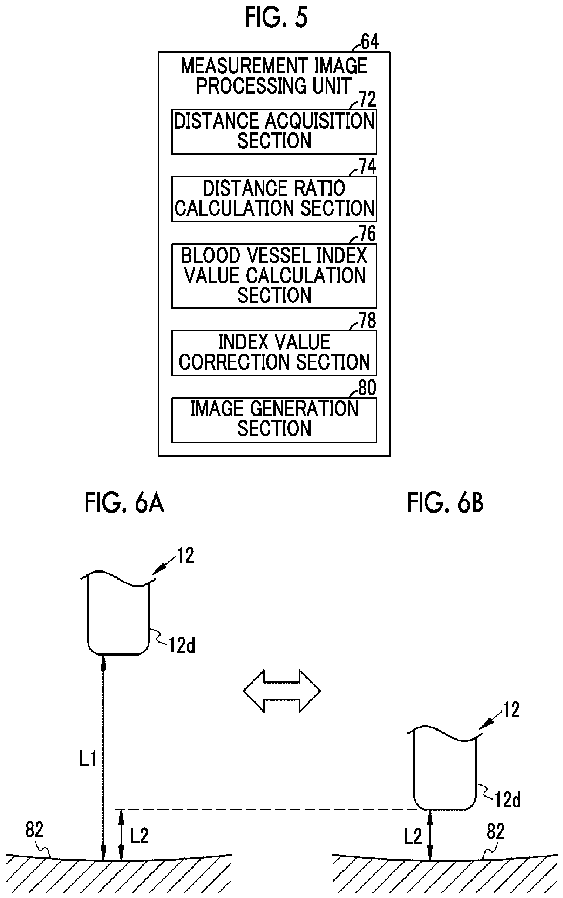

The measurement image processing unit 64 measures a blood vessel index value obtained by indexing (digitizing) the blood vessel indicated by the image signal received from the image processing switching unit 60. As shown in Fig. 5, a distance acquisition section 72, a distance ratio calculation section 74, a blood vessel index value calculation section 76, an index value correction section 78, and an image generation section 80 are provided in the measurement image processing unit 64. The image signal from the image processing switching unit 60 is received by the distance acquisition section 72, the blood vessel index value calculation section 76, and the image generation section 80.

As shown in Figs. 6A and 6B, the distance acquisition section 72 acquires the observation distance between the distal end portion 12d of the endoscope 12 and the surface of an observation target 82 from the image signal received from the image processing switching unit 60. In the present embodiment, an example will be described in which change from the non-magnified observation to the magnified observation is made by doctor's operation using the zoom operation unit 12g. Fig. 6A shows a non-magnified observation distance L1 that is an observation distance at the time of non-magnified observation. Fig. 6B shows a magnified observation distance L2 that is an observation distance at the time of magnified observation.

The distance acquisition section 72 receives an imaging magnification change instruction from the zoom operation unit 12g, and acquires, as the non-magnified observation distance L1, an observation distance in the event that the change of the imaging magnification is started based on the imaging magnification change instruction. In addition, the distance acquisition section 72 acquires, as the magnified observation distance L2, an observation distance in the event that the change of the imaging magnification is ended based on the imaging magnification change instruction. For example, the non-magnified observation distance L1 is set to 20 (mm), and the magnified observation distance L2 is set to 5 (mm). The non-magnified observation distance L1 corresponds to a "first observation distance" of the present invention, and the magnified observation distance L2 corresponds to a "second observation distance" of the present invention. The magnified observation distance L2 is also referred to as a specific observation distance. The distance acquisition section 72 transmits the acquired non-magnified observation distance L1 and the acquired magnified observation distance L2 to the distance ratio calculation section 74.

The observation distance can be acquired by an exposure amount obtained from an image signal, frequency analysis of an image signal, or the like. In the case of acquiring the observation distance from the exposure amount, the non-magnified observation distance L1 is acquired based on the exposure amount in the event that the change of the imaging magnification is started, and the magnified observation distance L2 is acquired based on the exposure amount in the event that the change of the imaging magnification is ended. In the case of acquiring the observation distance by frequency analysis, the non-magnified observation distance L1 is acquired by performing frequency analysis on the image signal in the event that the change of the imaging magnification is started, and the magnified observation distance L2 is acquired by performing frequency analysis on the image signal in the event that the change of the imaging magnification is ended.

The distance ratio calculation section 74 calculates a first distance ratio D as a distance ratio indicating the ratio between the non-magnified observation distance L1 and the magnified observation distance L2. The first distance ratio D is set to D = L1/L2. In the present embodiment, since L1 = 20 (mm) and L2 = 5 (mm), the first distance ratio D is "4". The distance ratio calculation section 74 transmits the calculated first distance ratio D to the index value correction section 78.

The blood vessel index value calculation section 76 calculates a blood vessel index value of a blood vessel indicated by the image signal received from the image processing switching unit 60. Blood vessel index values are the number of blood vessels of the observation target indicated by the image signal, a thickness (or a maximum blood vessel thickness that is the maximum value of the thickness of the blood vessel), a length (or an average blood vessel length that is the average value of the length of the blood vessel), a difference in height, an inclination, an area, a density (proportion of blood vessels in unit area), a depth, a gap, and the like. The blood vessel index value calculation section 76 calculates at least one or more of these blood vessel index values. For example, a blood vessel density, an average blood vessel length, and a maximum blood vessel thickness are calculated as blood vessel index values.

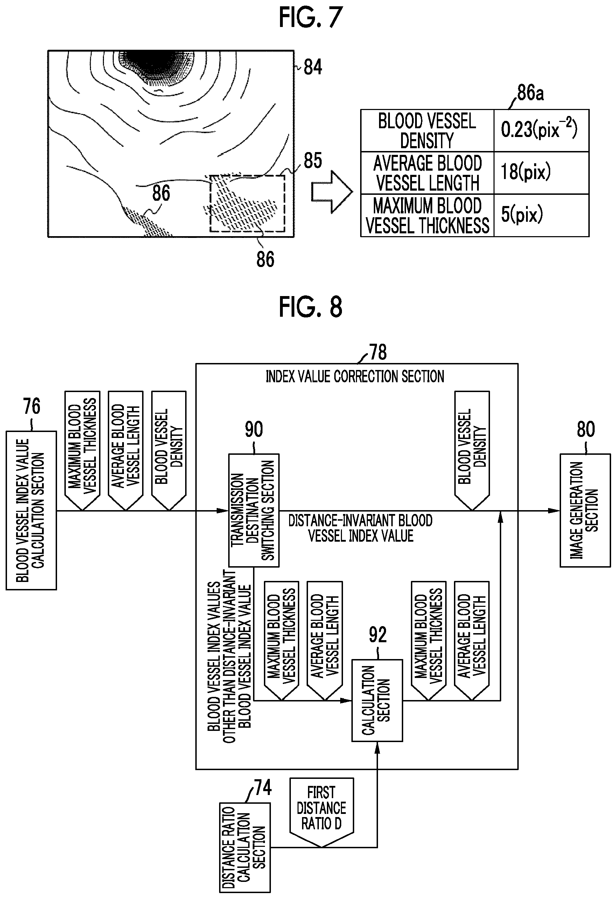

In the present embodiment, the blood vessel index value calculation section 76 calculates a blood vessel index value 86a of a blood vessel 86 within a region of interest (ROI) 85, which is set for an image signal 84, from the image signal 84 acquired at the non-magnified observation distance L1 (refer to Fig. 7) . Among the blood vessel index values 86a of the non-magnified observation distance L1 calculated by the blood vessel index value calculation section 76, the average blood vessel length and the maximum blood vessel thickness are indicated by the number of pixels (pixel: pix) indicating blood vessels on the screen, and the density of blood vessels is indicated by pix⁻².

As shown in Fig. 7, in the case of calculating the density of blood vessels, the blood vessel index value calculation section 76 cuts out a region having a specific size (unit area) within the ROI 85 of the image signal 84 acquired at the non-magnified observation distance L1, and calculates a proportion of the blood vessel 86 occupied in all the pixels within the region. By performing this on all the pixels within the ROI 85, the blood vessel index value calculation section 76 calculates the density of blood vessels within the ROI 85. In the case of calculating the average blood vessel length, the blood vessel index value calculation section 76 calculates the length of the blood vessel 86 in each pixel of the ROI 85, and calculates the average value of the length of the blood vessel 86 of each pixel. In the case of calculating the maximum blood vessel thickness, the blood vessel index value calculation section 76 calculates the thickness of the blood vessel 86 of each pixel of the ROI 85, and calculates the maximum value of the thickness of the blood vessel 86 of each pixel. In the present embodiment, the density of blood vessels of the non-magnified observation distance L1 is set to 0.23 (pix⁻²), the average blood vessel length is set to 18 (pix), and the maximum blood vessel thickness is set to 5 (pix). The blood vessel index value calculation section 76 transmits the calculated blood vessel index value 86a of the non-magnified observation distance L1 to the index value correction section 78.

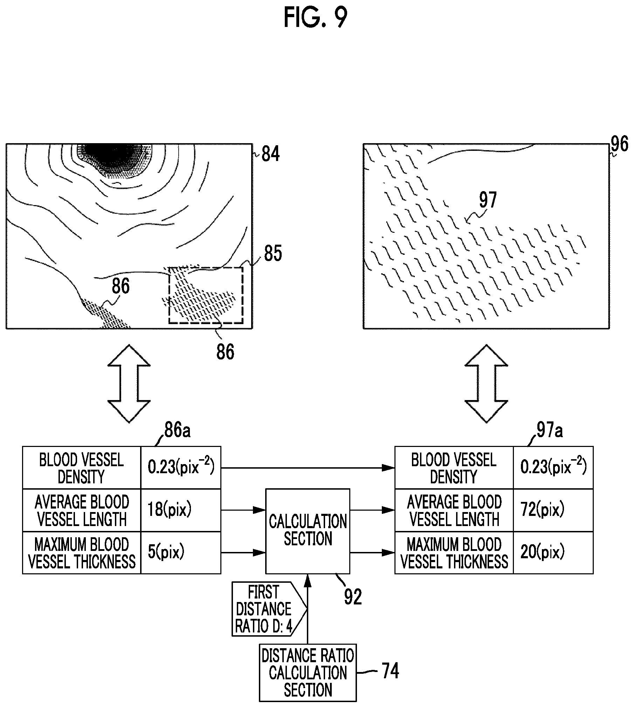

The index value correction section 78 acquires a blood vessel index value 97a of the magnified observation distance L2 using the blood vessel index value 86a of the non-magnified observation distance L1 (refer to Fig. 9). Specifically, the index value correction section 78 corrects the blood vessel index value 86a of the non-magnified observation distance L1 according to the first distance ratio D calculated by the distance ratio calculation section 74, thereby acquiring the blood vessel index value 97a of the magnified observation distance L2. As shown in Fig. 8, the index value correction section 78 includes a transmission destination switching section 90 and a calculation section 92.

The transmission destination switching section 90 receives the blood vessel index value 86a (a blood vessel density, an average blood vessel length, and a maximum blood vessel thickness) of the non-magnified observation distance L1 from the blood vessel index value calculation section 76, and switches a transmission destination so as to transmit the blood vessel index value 86a to the image generation section 80 in a case where the blood vessel index value 86a of the non-magnified observation distance L1 is a distance-invariant blood vessel index value and to transmit the blood vessel index value 86a to the calculation section 92 in a case where the blood vessel index value 86a of the non-magnified observation distance L1 is a blood vessel index value other than the distance-invariant blood vessel index value. The blood vessel index value 86a of the non-magnified observation distance L1 received from the blood vessel index value calculation section 76 includes a distance-invariant blood vessel index value and a blood vessel index value other than the distance-invariant blood vessel index value. The distance-invariant blood vessel index value is not changed even in a case where the length or thickness of the blood vessel in an image is changed due to a change in observation distance due to change from non-magnified observation to magnified observation. That is, the distance-invariant blood vessel index value is a blood vessel index value that is invariant with respect to the observation distance.

For example, the distance-invariant blood vessel index value is the density of blood vessels. The proportion occupied by the blood vessel 86 within the ROI 85 of the image signal 84 acquired at the non-magnified observation distance L1 is the same as the proportion occupied by a blood vessel 97 within an image signal 96 acquired at the magnified observation distance L2 in a case where magnified observation, in which the observation target in the ROI 85 is magnified, is performed (refer to Fig. 9). Therefore, the value of the blood vessel density at the non-magnified observation distance L1 is the same as that at the magnified observation distance L2.

In contrast, regarding the length of the blood vessel, the length of the blood vessel 97 of the image signal 96 acquired at the magnified observation distance L2 is larger than the length of the blood vessel 86 of the image signal 84 acquired at the non-magnified observation distance L1 (refer to Fig. 9). Therefore, the value of the average blood vessel length at the magnified observation distance L2 is larger than that at the non-magnified observation distance L1. Regarding the thickness of the blood vessel, the thickness of the blood vessel 97 of the image signal 96 acquired at the magnified observation distance L2 is larger than the thickness of the blood vessel 86 of the image signal 84 acquired at the non-magnified observation distance L1 (refer to Fig. 9). Therefore, the value of the maximum blood vessel thickness at the magnified observation distance L2 is larger than that at the non-magnified observation distance L1. In the present embodiment, therefore, the blood vessel density among the blood vessel density, the average blood vessel length, and the maximum blood vessel thickness is set as the distance-invariant blood vessel index value. Then, the transmission destination switching section 90 transmits the blood vessel density, among the blood vessel index values 86a of the non-magnified observation distance L1, to the image generation section 80, and transmits the average blood vessel length and the maximum blood vessel thickness to the calculation section 92 (refer to Fig. 8).

As shown in Fig. 9, the calculation section 92 corrects the average blood vessel length and the maximum blood vessel thickness of the non-magnified observation distance L1 by performing calculation using the average blood vessel length and the maximum blood vessel thickness other than the distance-invariant blood vessel index value, among the blood vessel index values 86a of the non-magnified observation distance L1 received from the transmission destination switching section 90, and the first distance ratio D received from the distance ratio calculation section 74, thereby calculating the average blood vessel length and the maximum blood vessel thickness of the magnified observation distance L2.

Specifically, the calculation section 92 calculates the average blood vessel length of the magnified observation distance L2 by multiplying the average blood vessel length of the non-magnified observation distance L1 by the first distance ratio D. In the present embodiment, since the average blood vessel length of the non-magnified observation distance L1 is 18 (pix) and the first distance ratio D is "4", the average blood vessel length of the magnified observation distance L2 is 72 (pix). Similarly, the calculation section 92 calculates the maximum blood vessel thickness of the non-magnified observation distance L2 by multiplying the maximum blood vessel thickness of the non-magnified observation distance L1 by the first distance ratio D. In the present embodiment, since the maximum blood vessel thickness of the non-magnified observation distance L1 is 5 (pix), the maximum blood vessel thickness of the magnified observation distance L2 is 20 (pix). The calculation section 92 transmits the calculated average blood vessel length and maximum blood vessel thickness of the magnified observation distance L2 to the image generation section 80.

The blood vessel index value 97a of the magnified observation distance L2 obtained by correcting the blood vessel index value 86a of the non-magnified observation distance L1 according to the first distance ratio D may be different from the blood vessel index value of the magnified observation distance L2 calculated based on the blood vessel 97 of the image signal 96 acquired at the magnified observation distance L2.

Specifically, in the case of magnified observation, it is difficult to acquire a blood vessel image with high resolution since even the slight movement of the endoscope 12 or the observation target influences the visibility of blood vessels in the image. Accordingly, in the image signal 96 acquired at the magnified observation distance L2 at the time of magnified observation, the resolution of the blood vessel 97 is not sufficient and the blood vessel 97 is easily blurred. For this reason, it is difficult to accurately calculate the blood vessel index value in many cases. In contrast, in the case of non-magnified observation, even assuming that the endoscope 12 or the observation target moves slightly, the blood vessel image can be acquired with high resolution. For this reason, the blood vessel index value 97a of the magnified observation distance L2 acquired based on the image signal 84 acquired at the non-magnified observation distance L1 is more accurate than the blood vessel index value of the magnified observation distance L2 calculated from the image signal 96 acquired at the magnified observation distance L2. For example, in a case where the blood vessel index value of the blood vessel 97 is calculated from the image signal 96 acquired at the magnified observation distance L2, the blood vessel density, the average blood vessel length, and the maximum blood vessel thickness that are blood vessel index values of the magnified observation distance L2 are 0.24 (pix⁻²), 73 (pix), and 21 (pix), respectively. Therefore, as shown in Fig. 9, these become different values from the blood vessel index value 97a of the magnified observation distance L2 calculated by the calculation section 92.

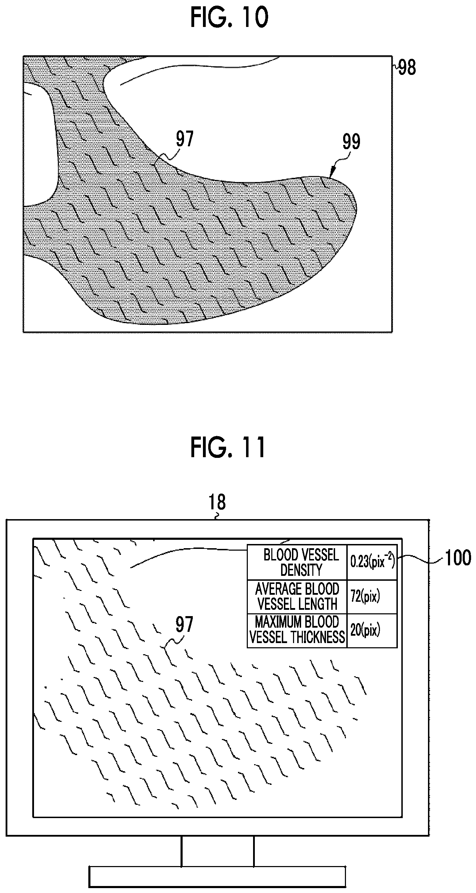

The image generation section 80 generates a special image signal based on the image signal and the blood vessel index value. An image using a special image signal is a special image. For example, the image generation section 80 generates a base image signal by performing color conversion processing, color enhancement processing, and structure enhancement processing on the image signal. Then, the image generation section 80 generates a special image signal by overlapping information based on the blood vessel index value with respect to the base image signal. In the overlapping process, the image generation section 80 performs coloring processing on the base image signal in accordance with the blood vessel index value, thereby displaying a region, in which the blood vessel index value is equal to or larger than a predetermined value, in a pseudo color. As a result, the image generation section 80 acquires a pseudo color special image signal. For example, in a special image signal 98 shown in Fig. 10, a region 99 where the blood vessel index value is equal to or larger than a predetermined value is displayed in a pseudo color. The image generation section 80 may display the pseudo color region 99 in a different color according to the blood vessel index value. In addition, the image generation section 80 may generate a special image by overlapping information, which indicates the blood vessel index value itself, with respect to the base image signal. For example, as shown in Fig. 11, the image generation section 80 may display information indicating the blood vessel index value in an upper right region 100 or the like on the monitor 18.

The video signal generation unit 66 converts the image signal received from the normal image processing unit 62 or the measurement image processing unit 64 into a video signal for displaying as an image that can be displayed on the monitor 18, and outputs the video signal to the monitor 18 in a sequential manner. Accordingly, the monitor 18 displays a normal image in a case where a normal image signal is input, and displays a special image in a case where a special image signal is input.

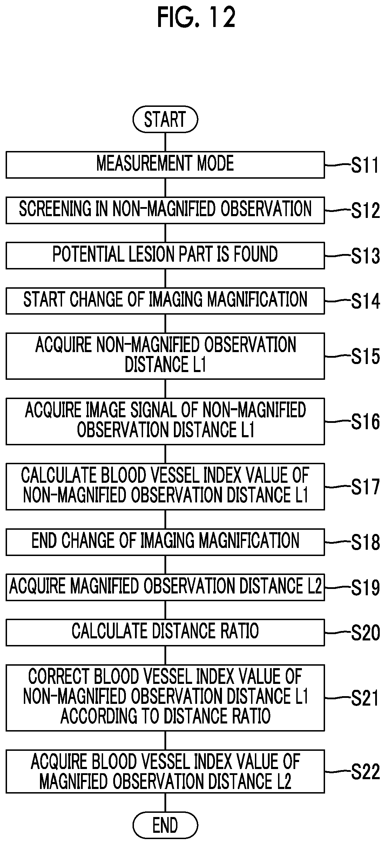

Next, the operation of the present invention will be described with reference to the flowchart shown in Fig. 12. First, the mode selector switch 12f is operated by the doctor, the observation mode is set to the measurement mode (S11), and screening is performed by non-magnified observation (S12). In the event that a part that is likely to be a lesion (hereinafter, referred to as a potential lesion part), such as a brownish area or rubor, is found at the time of screening (S13), the doctor operates the zoom operation unit 12g to start the change of the imaging magnification in order to observe the potential lesion part in an enlarged manner (S14).

In the event that the change of the imaging magnification is started, the distance acquisition section 72 acquires the non-magnified observation distance L1 (S15). The blood vessel index value calculation section 76 acquires the image signal 84 of the non-magnified observation distance L1 from the image processing switching unit 60 (S16), and calculates the blood vessel index value 86a of the non-magnified observation distance L1 from the image signal 84 (S17) . The blood vessel index value 86a of the non-magnified observation distance L1 that is calculated by the blood vessel index value calculation section 76 is at least one or more of a blood vessel density, a blood vessel length (or an average blood vessel length), a blood vessel thickness (or a maximum blood vessel thickness), the number of blood vessels, a difference in height between blood vessels, a blood vessel inclination, and a blood vessel depth. Then, in the event that the potential lesion part is magnified and observed, the doctor stops the operation of the zoom operation unit 12g to end the change of the imaging magnification (S18).

In the event that the change of the imaging magnification is ended, the distance acquisition section 72 acquires the magnified observation distance L2 (S19). The distance ratio calculation section 74 calculates the first distance ratio D as a distance ratio from the non-magnified observation distance L1 and the magnified observation distance L2 that have been acquired by the distance acquisition section 72 (S20). The index value correction section 78 corrects the blood vessel index value 86a of the non-magnified observation distance L1 according to the first distance ratio D (S21), and acquires the blood vessel index value 97a of the magnified observation distance L2 (S22).

As described above, in the present invention, since the blood vessel index value 97a of the magnified observation distance L2 is acquired by correcting the blood vessel index value 86a of the non-magnified observation distance L1 according to the first distance ratio D calculated by using the non-magnified observation distance L1 and the magnified observation distance L2, it is possible to acquire the blood vessel index value of the specific observation distance used for the predetermined diagnostic criteria.

In the image signal 96 acquired at the magnified observation distance L2, the blood vessel 97 may appear blurred. In this case, it is difficult for the blood vessel index value calculation section 76 to accurately calculate the blood vessel index value of the magnified observation distance L2. In contrast, in the present invention, the blood vessel index value calculation section 76 calculates the blood vessel index value 86a of the non-magnified observation distance L1 from the high resolution image signal 84 acquired at the non-magnified observation distance L1, and the index value correction section 78 acquires the blood vessel index value 97a of the magnified observation distance L2 by correcting the blood vessel index value 86a of the non-magnified observation distance L1, which has been calculated by the blood vessel index value calculation section 76, according to the first distance ratio D calculated by the distance ratio calculation section 74. Therefore, the blood vessel index value of the magnified observation distance L2 is accurate.

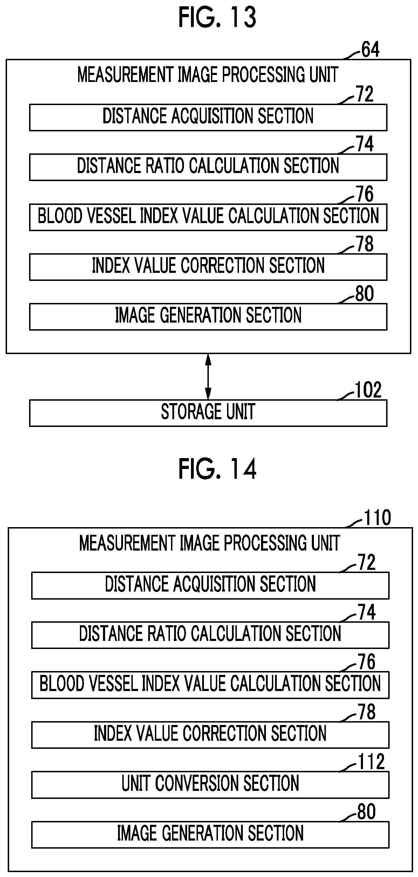

In the embodiment described above, the index value correction section 78 receives the blood vessel index value 86a of the non-magnified observation distance L1 from the blood vessel index value calculation section 76 and corrects the blood vessel index value 86a of the non-magnified observation distance L1 according to the first distance ratio D calculated by the distance ratio calculation section 74, thereby acquiring the blood vessel index value 97a of the magnified observation distance L2. However, the blood vessel index value 86a of the non-magnified observation distance L1 may be stored in a storage unit 102 shown in Fig. 13, and the blood vessel index value 97a of the magnified observation distance L2 may be acquired by correcting the blood vessel index value 86a stored in the storage unit 102 according to the first distance ratio D. The storage unit 102 sequentially stores the blood vessel index value 86a of the non-magnified observation distance L1 in a case where the blood vessel index value calculation section 76 calculates the blood vessel index value 86a of the non-magnified observation distance L1 during the non-magnified observation. The storage unit 102 may be provided in the processor device 16, or may be an external device connected to the processor device 16.

Thus, by storing the blood vessel index value 86a of the non-magnified observation distance L1 calculated by the blood vessel index value calculation section 76 during the non-magnified observation in the storage unit 102, the blood vessel index value of the magnified observation distance L2 can be acquired by correcting the blood vessel index value other than the blood vessel index value of the non-magnified observation distance L1 calculated by the blood vessel index value calculation section 76 in the event that the change of the imaging magnification is started according to the first distance ratio D.

The storage unit 102 may further store the first distance ratio D in a case where the distance ratio calculation section 74 calculates the first distance ratio D. In this case, a step of calculating the first distance ratio D by the distance ratio calculation section 74 is omitted.

In the above embodiment, an example has been described in which change from the non-magnified observation to the magnified observation is made by doctor' s operation using the zoom operation unit 12g. However, instead of this, the present invention can also be applied to a case in which change from the magnified observation to the non-magnified observation is made. In this case, based on the imaging magnification change instruction from the zoom operation unit 12g, the distance acquisition section 72 acquires the observation distance in the event that the change of the imaging magnification is started, as the magnified observation distance L2 (first observation distance), and acquires the observation distance in the event that the change of the imaging magnification is ended, as the non-magnified observation distance L1 (second observation distance). The distance ratio calculation section 74 sets the first distance ratio D to D = L2/L1. The blood vessel index value calculation section 76 calculates the blood vessel index value of the magnified observation distance L2 from the image signal acquired at the magnified observation distance L2. The index value correction section 78 corrects the blood vessel index value of the magnified observation distance L2 calculated by the blood vessel index value calculation section 76 according to the first distance ratio D calculated by the distance ratio calculation section 74, thereby acquiring the blood vessel index value of the non-magnified observation distance L1.

The unit of the blood vessel index value of the magnified observation distance L2 (specific observation distance) is expressed by the number of pixels (pix). However, the unit of the blood vessel index value of the magnified observation distance L2 (specific observation distance) may be converted into a specific unit different from the number of pixels.

In this case, as shown in Fig. 14, in addition to each component of the measurement image processing unit 64, a unit conversion section 112 that converts the number of pixels of the blood vessel index value into a specific unit is newly provided in a measurement image processing section 110. In the unit conversion section 112, a predetermined observation distance for unit conversion Lt and a blood vessel index value of a specific unit corresponding to 1 (pix) at the observation distance for unit conversion Lt are stored so as to be associated with each other. Then, in a case where the blood vessel index value 97a of the magnified observation distance L2 expressed by the number of pixels is acquired by the index value correction section 78, the unit conversion section 112 calculates a conversion ratio T indicating the ratio between the magnified observation distance L2 and the observation distance for unit conversion Lt stored in the unit conversion section 112. Then, the unit conversion section 112 converts the blood vessel index value 97a of the magnified observation distance L2 from the number of pixels to a specific unit by performing calculation based on the conversion ratio T and the blood vessel index value 97a of the magnified observation distance L2. The specific unit is, for example, millimeter (mm). Specifically, in a case where the magnified observation distance L2 is 5 (mm), the maximum blood vessel thickness of the magnified observation distance L2 is 20 (pix), the observation distance for unit conversion Lt is 10 (mm), and 1 (pix) at the observation distance for unit conversion Lt is 0.1 (mm), the unit conversion section 112 calculates the conversion ratio T as T = L2/Ls = 0.5. The unit conversion section 112 multiplies the conversion ratio T (= 0.5) by the maximum blood vessel thickness 20 (pix) of the magnified observation distance L2 expressed by the number of pixels (0.5 x 20). As a result, the maximum blood vessel thickness of the magnified observation distance L2 is converted into 1 (mm).

In the embodiment described above, the first observation distance is acquired and the second observation distance is acquired as a specific observation distance, the first distance ratio D indicating the ratio between the first observation distance and the second observation distance is calculated, and the blood vessel index value of the first observation distance is corrected according to the first distance ratio D, thereby acquiring the blood vessel index value of the second observation distance. However, a predetermined reference observation distance Ls as a specific observation distance may be set, a second distance ratio Ds indicating the ratio between the first observation distance and the reference observation distance Ls may be calculated, and the blood vessel index value of the first observation distance may be corrected according to the second distance ratio Ds, thereby acquiring the blood vessel index value of the reference observation distance Ls.

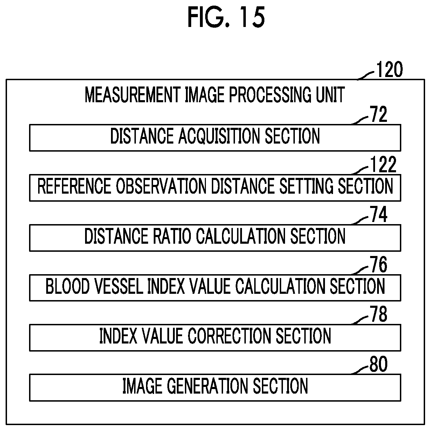

As shown in Fig. 15, in addition to each component of the measurement image processing unit 64, a reference observation distance setting section 122 that sets the reference observation distance Ls is newly provided in a measurement image processing section 120. The reference observation distance setting section 122 is connected to a user interface, such as the console 19, and transmits the reference observation distance Ls to the distance ratio calculation section 74 in a case where the reference observation distance Ls is input from the console 19. The reference observation distance Ls is, for example, 10 (mm).

The distance ratio calculation section 74 calculates the second distance ratio Ds (= L1/Ls) indicating the ratio between the non-magnified observation distance L1 and the reference observation distance Ls. The index value correction section 78 corrects the blood vessel index value of the non-magnified observation distance L1 by multiplying the blood vessel index value of the non-magnified observation distance L1 by the second distance ratio Ds, thereby acquiring the blood vessel index value of the reference observation distance Ls. For example, since the second distance ratio Ds is Ds = 20/10 = 2 and the average blood vessel length of the non-magnified observation distance L1 is 18 (pix), the average blood vessel length of the reference observation distance Ls is 36 (pix). In addition, since the maximum blood vessel thickness of the non-magnified observation distance L1 is 5 (pix), the maximum blood vessel thickness of the reference observation distance Ls is 10 (pix).

Thus, the measurement image processing section 120 can acquire the blood vessel index value of the reference observation distance Ls even assuming that the first observation distance is not adjusted to the reference observation distance Ls by correcting the blood vessel index value of the first observation distance using the second distance ratio Ds that is calculated by using the first observation distance and the reference observation distance Ls. The reference observation distance Ls is preferably an observation distance at which diagnostic criteria according to biological information, such as a blood vessel index value, are determined. In this case, since the blood vessel index value of the accurate reference observation distance Ls can be acquired, a more reliable diagnosis is possible. In addition, the reference observation distance Ls may be preset in the reference observation distance setting section 122. The preset reference observation distance Ls may be changed by the console 19 or the like.

In the case of acquiring the blood vessel index value of the reference observation distance Ls by correcting the blood vessel index value of the first observation distance according to the second distance ratio Ds, the unit conversion section 112 may store the blood vessel index value of a specific unit corresponding to 1 (pix) at the reference observation distance Ls. Accordingly, since the calculation of the conversion ratio T is omitted, the unit of the blood vessel index value of the reference observation distance Ls can be easily converted into a specific unit different from the number of pixels.

The distance ratio calculation section 74 may calculate a ratio between the magnified observation distance L2 and the reference observation distance Ls as the second distance ratio Ds. In this case, the index value correction section 78 can acquire the blood vessel index value of the reference observation distance Ls by correcting the blood vessel index value of the magnified observation distance L2 according to the second distance ratio Ds.

### [Second embodiment]

In a second embodiment, the observation target is illuminated using a laser light source and a phosphor instead of the LEDs 20a to 20d of four colors shown in the first embodiment. Others are the same as in the first embodiment.

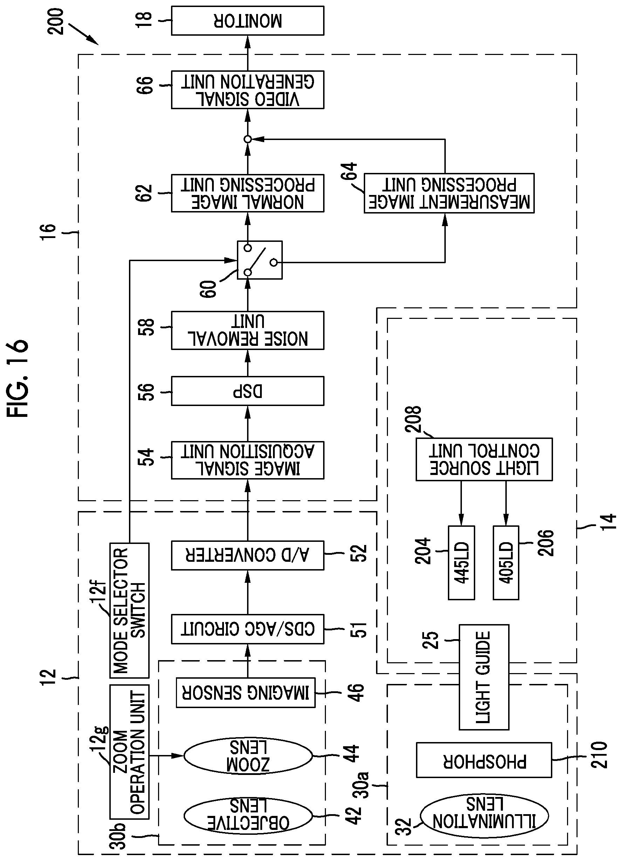

As shown in Fig. 16, in an endoscope system 200 of the second embodiment, instead of the LEDs 20a to 20d of four colors shown in the first embodiment, a blue laser light source (denoted as "445LD" in Fig. 16) 204 that emits blue laser light having a center wavelength of 445+10 nm and a blue-violet laser light source (denoted as "405LD" in Fig. 16) 206 that emits blue-violet laser light having a center wavelength of 405+10 nm are provided in the light source device 14. Emission from semiconductor light emitting elements of the light sources 204 and 206 is individually controlled by a light source control unit 208. In addition, the light amount ratio between light emitted from the blue laser light source 204 and light emitted from the blue-violet laser light source 206 can be freely changed by the light source control unit 208.

The light source control unit 208 drives the blue laser light source 204 in the case of a normal mode. On the other hand, in the case of a measurement mode, both the blue laser light source 204 and the blue-violet laser light source 206 are driven, and the emission ratio of blue laser light is controlled to be larger than the emission ratio of blue-violet laser light. The laser light emitted from each of the light sources 204 and 206 is incident on the light guide 25 through optical members such as a condensing lens, an optical fiber, and a multiplexer (none is shown).

It is preferable that the half-width of blue laser light or blue-violet laser light is set to about ±10 nm. As the blue laser light source 204 and the blue-violet laser light source 206, a broad area type InGaN-based laser diode can be used, and an InGaNAs-based laser diode or a GaNAs-based laser diode can be used. As the light sources, a structure using a light emitter, such as a light emitting diode, may be used.

In addition to the illumination lens 32, a phosphor 210 on which blue laser light or blue-violet laser light from the light guide 25 is incident is provided in the illumination optical system 30a. The phosphor 210 is excited by the blue laser light and emits fluorescence. A part of the blue laser light is transmitted without exciting the phosphor 210. The blue-violet laser light is transmitted without exciting the phosphor 210. The light emitted from the phosphor 210 illuminates the inside of the body of the observation target through the illumination lens 32.

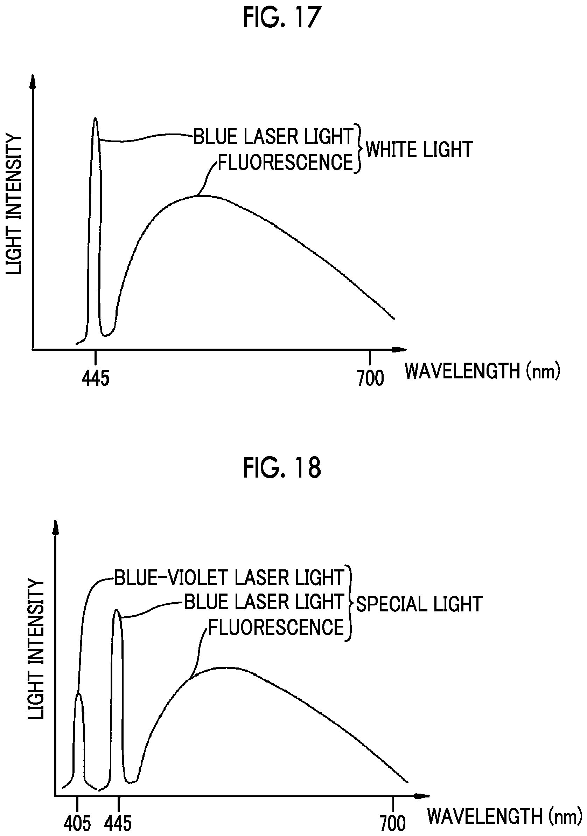

In the normal mode, the blue laser light is mainly incident on the phosphor 210. Accordingly, the observation target is illuminated with white light obtained by combining the blue laser light and the fluorescence that is excited and emitted from the phosphor 210 by the blue laser light as shown in Fig. 17. On the other hand, in the measurement mode, both the blue-violet laser light and the blue laser light are incident on the phosphor 210. Accordingly, the observation target is illuminated with special light obtained by combining the blue-violet laser light, the blue laser light, and the fluorescence that is excited and emitted from the phosphor 210 by the blue laser light as shown in Fig. 18.

As the phosphor 210, it is preferable to use a phosphor configured to include a plurality of kinds of phosphors (for example, a YAG-based phosphor or a phosphor, such as BAM (BaMgAl₁₀O₁₇)) that absorb a part of blue laser light and are excited to emit green to yellow light beams. Assuming that the semiconductor light emitting element is used as the excitation light source of the phosphor 210 as in this configuration example, high-density white light can be obtained with high luminous efficiency. Therefore, it is possible to easily adjust the intensity of white light and to suppress the change in color temperature and chromaticity of white light.

### [Third embodiment]

In a third embodiment, the observation target is illuminated using a broadband light source, such as a xenon lamp, and a rotary filter instead of the LEDs 20a to 20d of four colors shown in the first embodiment. In addition, the observation target is imaged using a monochrome imaging sensor instead of the color imaging sensor 46. Others are the same as in the first embodiment.

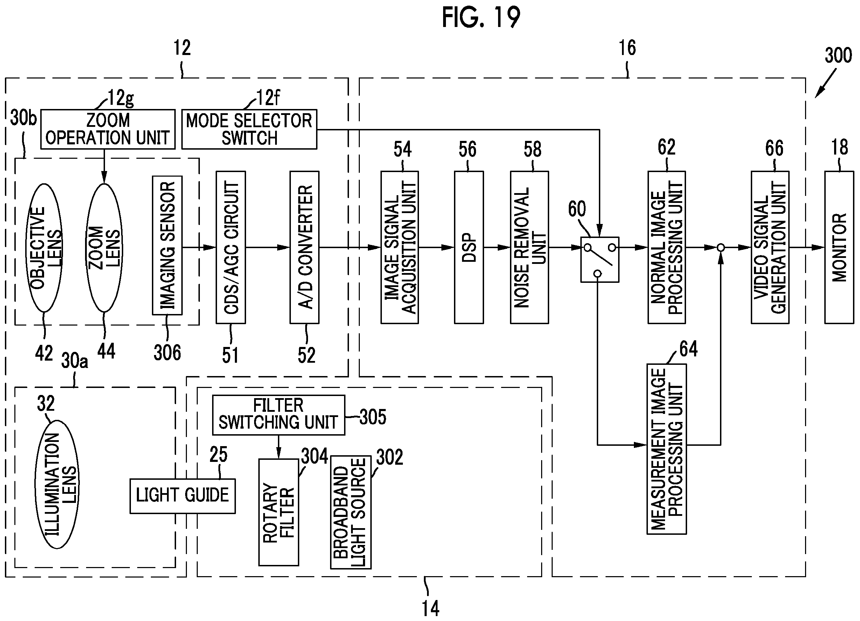

As shown in Fig. 19, in an endoscope system 300 of the third embodiment, instead of the LEDs 20a to 20d of four colors shown in the first embodiment, a broadband light source 302, a rotary filter 304, and a filter switching unit 305 are provided in the light source device 14. In addition, instead of the color imaging sensor 46, a monochrome imaging sensor 306 in which no color filter is provided is provided in the imaging optical system 30b.

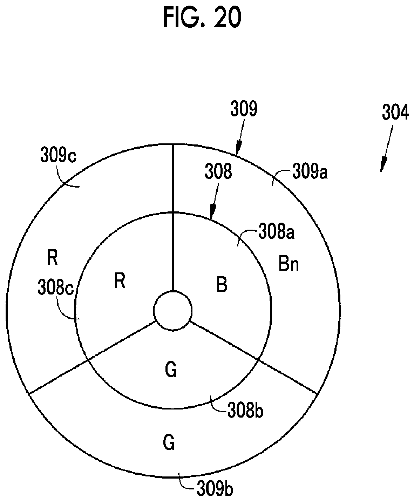

The broadband light source 302 is a xenon lamp, a white LED, or the like, and emits white light having a wavelength range from blue to red. The rotary filter 304 includes a normal mode filter 308 provided on the inner side and a measurement mode filter 309 provided on the outer side (refer to Fig. 20). The filter switching unit 305 moves the rotary filter 304 in the radial direction. In a case where the doctor operates the mode selector switch 12f to set the normal mode, the filter switching unit 305 inserts the normal mode filter 308 of the rotary filter 304 in the optical path of white light. On the other hand, in the event that the doctor operates the mode selector switch 12f to set the measurement mode, the filter switching unit 305 inserts the measurement mode filter 309 of the rotary filter 304 in the optical path of white light.

As shown in Fig. 20, a B filter 308a that transmits blue light of the white light, a G filter 308b that transmits green light of the white light, and an R filter 308c that transmits red light of the white light are provided along the circumferential direction in the normal mode filter 308. Therefore, at the time of normal mode, the rotary filter 304 rotates to alternately emit the blue light, the green light, and the red light to the observation target.

A Bn filter 309a that transmits blue narrowband light having a specific wavelength of the white light, a G filter 309b that transmits green light of the white light, and an R filter 309c that transmits red light of the white light are provided along the circumferential direction in the measurement mode filter 309. Therefore, at the time of measurement mode, the rotary filter 304 rotates to alternately emit the blue narrowband light, the green light, and the red light to the observation target.

In the endoscope system 300, at the time of normal mode, the monochrome imaging sensor 306 images the observation target every time the observation target is illuminated with the blue light, the green light, and the red light. As a result, image signals of three colors of RGB are obtained. Then, based on the image signals of RGB colors, a normal image is generated using the same method as in the first embodiment described above.

On the other hand, at the time of measurement mode, the monochrome imaging sensor 306 images the observation target every time the observation target is illuminated with the blue narrowband light, the green light, and the red light. As a result, a Bn image signal, a G image signal, and an R image signal are obtained. Based on the Bn image signal, the G image signal, and the R image signal, a special image is generated. Thus, in order to generate a special image, a Bn image signal is used instead of the B image signal. Other than that, the special image is generated using the same method as in the first embodiment.

A blood vessel index value may be selected by a user interface, such as the console 19, and the blood vessel index value calculation section 76 may calculate a blood vessel index value for the selected blood vessel index value . The blood vessel index value calculation section 76 may calculate all blood vessel index values, that is, the number of blood vessels, a thickness (or a maximum blood vessel thickness), a length (or an average blood vessel length), a difference in height, an inclination, an area, a density, a depth, and a blood vessel gap.

The functions of the distance acquisition section 72, the distance ratio calculation section 74, the image signal acquisition unit 54, the blood vessel index value calculation section 76, and the index value correction section 78 may be stored in the ROM as a program, and a control unit may be made to function as each of the units based on the program.

## Claims

1. A processor device for an endoscope (12), comprising:
a distance acquisition unit (72) that acquires a first observation distance (L1) that is a non-magnified observation distance between a distal end portion (12d) of the endoscope (12) and an observation target (82) and at least one specific observation distance (L2) that is a magnified observation distance;
a distance ratio calculation unit (74) that calculates a distance ratio (D) between the first observation distance (L1) and the specific observation distance (L2);
an image signal acquisition unit (54) that acquires an image signal (84) by imaging the observation target (82) with the endoscope (12) at the first observation distance (L1);
a blood vessel index value calculation unit (76) that calculates a first blood vessel index value (86a) of a blood vessel of the observation target (82) by digitizing information regarding a blood vessel from the image signal (84) acquired at the first observation distance (L1);
**characterized in that**:
the first blood vessel index value (86a) comprises distance-invariant blood vessel index values and distance variant blood vessel index values; and that
the processor device further comprises:
an index value correction unit (78) configured to calculate a second blood vessel index value (97a) at the specific observation distance (L2);
wherein the index value correction unit (78) is configured to receive the first blood vessel index value (86a) of the non-magnified observation distance (L1) and to correct the distance variant blood vessel index values of the first blood vessel index value (86a) according to the distance ratio and not change the distance-invariant blood vessel index values of the first blood vessel index value (86a).

2. The processor device for an endoscope (12) according to claim 1, wherein the distance-invariant blood vessel index value is a density.

3. The processor device for an endoscope (12) according to any one of claims 1 to 2, further comprising:
a storage unit that stores the first blood vessel index value (86a) .

4. The processor device for an endoscope (12) according to claim 3,
wherein the storage unit further stores the distance ratio.

5. The processor device for an endoscope (12) according to any one of claims 1 to 4, further comprising:
a unit conversion unit that converts the second blood vessel index value (97a)from a number of pixels to a specific unit.

6. The processor device for an endoscope (12) according to any one of claims 1 to 5,
wherein the distance acquisition unit (72) further acquires a third observation distance as the specific observation distance,
the distance ratio calculation unit (74) calculates, as a second distance ratio, the distance ratio in a case where the specific observation distance is set to the third observation distance, and
the index value correction unit (78) acquires a blood vessel index value of the third observation distance as the third blood vessel index value (97a) by correcting the distance variant blood vessel index values of the first blood vessel index value (86a) according to the second distance ratio.

7. An endoscope system comprising the processor device for an endoscope (12) according to claim and the endoscope (12), wherein the endoscope (12) further comprises:
an imaging optical system (30b) capable of changing an imaging magnification between the first observation distance and the second observation distance; and
an imaging magnification change operation unit (12g) for inputting an imaging magnification change instruction to change the imaging magnification to the imaging optical system,
wherein the distance acquisition unit (72) acquires the first observation distance and the second observation distance based on the imaging magnification change instruction.

8. The processor device for an endoscope (12) according to any one of claims 1 to 5, further comprising:
a reference observation distance setting unit that sets a predetermined reference observation distance,
wherein the distance ratio calculation unit (74) calculates, as a second distance ratio, the distance ratio in a case where the specific observation distance is set to the reference observation distance, and
the index value correction unit (78) acquires a blood vessel index value of the reference observation distance as the second blood vessel index value (97a) by correcting the distance variant blood vessel index values of the first blood vessel index value (86a) according to the second distance ratio.

9. A non-transitory computer readable medium comprising a control program for an endoscope (12) wherein when the control program is installed in a processor device for the endoscope (12), the program causing the processor device to function as:
a distance acquisition unit (72) that acquires a first observation distance (L1) that is a non-magnified observation distance between a distal end portion (12d) of the endoscope (12) and an observation target (82) and at least one specific observation distance (L2) that is a magnified observation distance;
a distance ratio calculation unit (74) that calculates a distance ratio (D) between the first observation distance (L1) and the specific observation distance (L2);
an image signal acquisition unit (54) that acquires an image signal (84) by imaging the observation target (82) with the endoscope (12) at the first observation distance (L1);
a blood vessel index value calculation unit (76) that calculates a first blood vessel index value (86a) of a blood vessel of the observation target (82) by digitizing information regarding a blood vessel from the image signal (84) acquired at the first observation distance (L1);
**characterized in that**:
the first blood vessel index value (86a) comprises distance-invariant blood vessel index values and distance variant blood vessel index values; and that
the program further causes the computer to function as:
an index value correction unit (78) configured to calculate a second blood vessel index value (97a) at the specific observation distance (L2);
wherein the index value correction unit (78) is configured to receive the first blood vessel index value (86a) of the non-magnified observation distance (L1) and to correct the distance variant blood vessel index values of the first blood vessel index value (86a) according to the distance ratio and not change the distance-invariant blood vessel index values of the first blood vessel index value (86a).

## Patentansprüche

1. Prozessorvorrichtung für ein Endoskop (12), die umfasst:
eine Distanzaufzeichnungseinheit (72), die eine erste Beobachtungsdistanz (L1) aufzeichnet, die eine nicht vergrößerte Beobachtungsdistanz zwischen einem distalen Endabschnitt (12d) des Endoskops (12) und einem Beobachtungsziel (82) ist, und mindestens eine spezifische Beobachtungsdistanz (L2), die eine vergrößerte Beobachtungsdistanz ist;
eine Distanzverhältnis-Berechnungseinheit (74), die ein Distanzverhältnis (D) zwischen der ersten Beobachtungsdistanz (L1) und der spezifischen Beobachtungsdistanz (L2) berechnet;
eine Bildsignalaufzeichnungseinheit (54), die ein Bildsignal (84) durch Abbilden des Beobachtungsziels (82) mit dem Endoskop (12) an der ersten Beobachtungsdistanz (L1) aufzeichnet;
eine Blutgefäß-Indexwertberechnungseinheit (76), die einen ersten Blutgefäß-Indexwert (86a) eines Blutgefäßes des Beobachtungsziels (82) durch Digitalisieren von Informationen im Zusammenhang mit einem Blutgefäß von dem Bildsignal (84), das an der ersten Beobachtungsdistanz (L1) aufgezeichnet wird, berechnet;
**dadurch gekennzeichnet, dass**:
der erste Blutgefäß-Indexwert (86a) distanzinvariante Blutgefäß-Indexwerte und distanzvariante Blutgefäß-Indexwerte umfasst; und dass
die Prozessorvorrichtung weiter umfasst:
eine Indexwert-Korrektureinheit (78), die dazu konfiguriert ist, einen zweiten Blutgefäß-Indexwert (97a) an der spezifischen Beobachtungsdistanz (L2) zu berechnen;
wobei die Indexwert-Korrektureinheit (78) dazu konfiguriert ist, den ersten Blutgefäß-Indexwert (86a) der nicht vergrößerten Beobachtungsdistanz (L1) zu empfangen und die distanzvarianten Blutgefäß-Indexwerte des ersten Blutgefäß-Indexwerts (86a) gemäß dem Distanzverhältnis zu korrigieren und die distanzinvarianten Blutgefäß-Indexwerte des ersten Blutgefäß-Indexwerts (86a) nicht zu ändern.

2. Prozessorvorrichtung für ein Endoskop (12) nach Anspruch 1,
wobei der distanzinvariante Blutgefäß-Indexwert eine Dichte ist.

3. Prozessorvorrichtung für ein Endoskop (12) nach einem der Ansprüche 1 und 2, das weiter umfasst:
eine Speicherungseinheit, die den ersten Blutgefäß-Indexwert (86a) speichert.

4. Prozessorvorrichtung für ein Endoskop (12) nach Anspruch 3,
wobei die Speicherungseinheit weiter das Distanzverhältnis speichert.

5. Prozessorvorrichtung für ein Endoskop (12) nach einem der Ansprüche 1 und 4, das weiter umfasst:
eine Einheitsumwandlungseinheit, die den zweiten Blutgefäß-Indexwert (97a) von einer Anzahl von Pixeln in eine spezifische Einheit umwandelt.

6. Prozessorvorrichtung für ein Endoskop (12) nach einem der Ansprüche 1 bis 5,
wobei die Distanzaufzeichnungseinheit (72) weiter eine dritte Beobachtungsdistanz als die spezifische Beobachtungsdistanz aufzeichnet,
die Distanzverhältnis-Berechnungseinheit (74) als ein zweites Distanzverhältnis das Distanzverhältnis in einem Fall berechnet, dass die spezifische Beobachtungsdistanz auf die dritte Beobachtungsdistanz eingestellt ist, und
die Indexwert-Korrektureinheit (78) einen Blutgefäß-Indexwert der dritten Beobachtungsdistanz als den dritten Blutgefäß-Indexwert (97a) durch Korrigieren der distanzvarianten Blutgefäß-Indexwerte des ersten Blutgefäß-Indexwerts (86a) gemäß dem zweiten Distanzverhältnis aufzeichnet.

7. Endoskopsystem, das die Prozessorvorrichtung für ein Endoskop (12) nach Anspruch 6 und das Endoskop (12) umfasst, wobei das Endoskop (12) weiter umfasst:
ein optisches Bildgebungssystem (30b), das in der Lage ist, eine Bildgebungsvergrößerung zwischen der ersten Beobachtungsdistanz und der zweiten Beobachtungsdistanz zu ändern; und
eine Bildgebungsvergrößerungs-Änderungsbetriebseinheit (12g) zum Eingeben einer Bildgebungsvergrößerungs-Änderungsanweisung zum Ändern der Bildgebungsvergrößerung zu dem optischen Bildgebungssystem,
wobei die Distanzaufzeichnungseinheit (72) die erste Beobachtungsdistanz und die zweite Beobachtungsdistanz basierend auf der Bildgebungsvergrößerungs-Änderungsanweisung aufzeichnet.

8. Prozessorvorrichtung für ein Endoskop (12) nach einem der Ansprüche 1 bis 5, das weiter umfasst:
eine Referenzbeobachtungsdistanz-Einstelleinheit, die eine vorbestimmte Referenzbeobachtungsdistanz einstellt,
wobei die Distanzverhältnis-Berechnungseinheit (74) als ein zweites Distanzverhältnis das Distanzverhältnis in einem Fall berechnet, dass die spezifische Beobachtungsdistanz auf die Referenzbeobachtungsdistanz eingestellt ist, und
die Indexwert-Korrektureinheit (78) einen Blutgefäß-Indexwert der Referenzbeobachtungsdistanz als den zweiten Blutgefäß-Indexwert (97a) durch Korrigieren der distanzvarianten Blutgefäß-Indexwerte des ersten Blutgefäß-Indexwerts (86a) gemäß dem zweiten Distanzverhältnis aufzeichnet.

9. Nichtflüchtiges computerlesbares Medium, das ein Steuerprogramm für ein Endoskop (12) umfasst, wobei, wenn das Steuerprogramm in einer Prozessorvorrichtung für das Endoskop (12) installiert ist, das Programm die Prozessorvorrichtung veranlasst zu funktionieren als:
eine Distanzaufzeichnungseinheit (72), die eine erste Beobachtungsentfernung (L1) aufzeichnet, die eine nicht vergrößerte Beobachtungsentfernung zwischen einem distalen Endabschnitt (12d) des Endoskops (12) und einem Beobachtungsziel (82) ist, und mindestens eine spezifische Beobachtungsentfernung (L2), die eine vergrößerte Beobachtungsentfernung ist;
eine Distanzverhältnis-Berechnungseinheit (74), die ein Distanzverhältnis (D) zwischen der ersten Beobachtungsdistanz (L1) und der spezifischen Beobachtungsdistanz (L2) berechnet;
eine Bildsignalaufzeichnungseinheit (54), die ein Bildsignal (84) durch Abbilden des Beobachtungsziels (82) mit dem Endoskop (12) an der ersten Beobachtungsdistanz (L1) aufzeichnet;
eine Blutgefäß-Indexwertberechnungseinheit (76), die einen ersten Blutgefäß-Indexwert (86a) eines Blutgefäßes des Beobachtungsziels (82) durch Digitalisieren von Informationen im Zusammenhang mit einem Blutgefäß aus dem Bildsignal (84), das an der ersten Beobachtungsdistanz (L1) aufgezeichnet wird, berechnet;
**dadurch gekennzeichnet, dass**:
der erste Blutgefäß-Indexwert (86a) distanzinvariante Blutgefäß-Indexwerte und distanzvariante Blutgefäß-Indexwerte umfasst; und dass
das Programm weiter den Computer veranlasst, zu funktionieren als: eine Indexwert-Korrektureinheit (78), die dazu konfiguriert ist, einen zweiten Blutgefäß-Indexwert (97a) an der spezifischen Beobachtungsdistanz (L2) zu berechnen;
wobei die Indexwert-Korrektureinheit (78) dazu konfiguriert ist, den ersten Blutgefäß-Indexwert (86a) der nicht vergrößerten Beobachtungsdistanz (L1) zu empfangen und die distanzvarianten Blutgefäß-Indexwerte des ersten Blutgefäß-Indexwerts (86a) gemäß dem Distanzverhältnis zu korrigieren und die distanzinvarianten Blutgefäß-Indexwerte des ersten Blutgefäß-Indexwerts (86a) nicht zu ändern.

## Revendications

1. Dispositif de processeur pour un endoscope (12), comprenant :
une unité d'acquisition de distance (72) qui permet d'obtenir une première distance d'observation (L1) qui est une distance d'observation non amplifiée entre une partie d'extrémité distale (12d) de l'endoscope (12) et une cible d'observation (82) et au moins une distance d'observation particulière (L2) qui est une distance d'observation amplifiée ;
une unité de calcul de rapport de distances (74) qui permet de calculer un rapport de distances (D) entre la première distance d'observation (L1) et la distance d'observation particulière (L2) ;
une unité d'acquisition de signal d'image (54) qui permet d'obtenir un signal d'image (84) par imagerie de la cible d'observation (82) avec l'endoscope (12) au niveau de la première distance d'observation (L1) ;
une unité de calcul de la valeur d'indice du vaisseau sanguin (76) qui permet de calculer une première valeur d'indice de vaisseau sanguin (86a) d'un vaisseau sanguin de la cible d'observation (82) par numérisation de données relatives à un vaisseau sanguin à partir d'un signal d'image (84) obtenu au niveau de la première distance d'observation (L1);
**caractérisé en ce que** :
la première valeur d'indice de vaisseau sanguin (86a) comprend des valeurs d'indice de vaisseau sanguin invariables en fonction de la distance et des valeurs d'indice de vaisseau sanguin variables en fonction de la distance ; et **en ce que**
le dispositif de processeur comprend en outre :
une unité de correction de la valeur d'indice (78) configurée pour calculer une deuxième valeur d'indice de vaisseau sanguin (97a) au niveau de la distance d'observation particulière (L2) ;
dans lequel l'unité de correction de la valeur d'indice (78) est configurée pour recevoir la première valeur d'indice de vaisseau sanguin (86a) de la distance d'observation non amplifiée (L1) et pour corriger les valeurs d'indice de vaisseau sanguin variables en fonction de la distance de la première valeur d'indice de vaisseau sanguin (86a) selon le rapport de distances et ne pas modifier les valeurs d'indice de vaisseau sanguin invariables en fonction de la distance de la première valeur d'indice de vaisseau sanguin (86a).

2. Dispositif de processeur pour un endoscope (12) selon la revendication 1,
dans lequel la valeur d'indice de vaisseau sanguin invariable en fonction de la distance est une densité.

3. Dispositif de processeur pour un endoscope (12) selon l'une quelconque des revendications 1 à 2, comprenant en outre :
une unité de stockage qui permet de stocker la première valeur d'indice de vaisseau sanguin (86a).

4. Dispositif de processeur pour un endoscope (12) selon la revendication 3,
dans lequel l'unité de stockage permet de stocker en outre le rapport de distances.

5. Dispositif de processeur pour un endoscope (12) selon l'une quelconque des revendications 1 à 4, comprenant en outre :
une unité de conversion d'unité qui permet de convertir la deuxième valeur d'indice de vaisseau sanguin (97a) d'un nombre de pixels en une unité particulière.

6. Dispositif de processeur pour un endoscope (12) selon l'une quelconque des revendications 1 à 5,
dans lequel l'unité d'acquisition de distance (72) permet d'obtenir en outre une troisième distance d'observation en tant que distance d'observation particulière,
l'unité de calcul de rapport de distances (74) permet de calculer, en tant que second rapport de distance, le rapport de distances dans un cas où la distance d'observation particulière est fixée à la troisième distance d'observation, et
l'unité de correction de la valeur d'indice (78) permet d'obtenir une valeur d'indice de vaisseau sanguin de la troisième distance d'observation en tant que troisième valeur d'indice de vaisseau sanguin (97a) par correction des valeurs d'indice de vaisseau sanguin variables en fonction de la distance de la première valeur d'indice de vaisseau sanguin (86a) selon le second rapport de distances.

7. Système d'endoscope comprenant le dispositif de processeur pour un endoscope (12) selon la revendication 6, et l'endoscope (12), dans lequel l'endoscope (12) comprend en outre :
un système optique d'imagerie (30b) capable de changer un grossissement d'imagerie entre la première distance d'observation et la deuxième distance d'observation ; et
une unité d'opération de changement de grossissement d'imagerie (12g) permettant d'entrer une instruction de changement de grossissement d'imagerie pour changer le grossissement d'imagerie dans le système optique d'imagerie,
dans lequel l'unité d'acquisition de distance (72) permet d'obtenir la première distance d'observation et la deuxième distance d'observation en fonction de l'instruction de changement de grossissement d'imagerie.

8. Dispositif de processeur pour un endoscope (12) selon l'une quelconque des revendications 1 à 5, comprenant en outre :
une unité de réglage de distance d'observation de référence qui permet de fixer une distance d'observation de référence prédéterminée,
dans lequel l'unité de calcul de rapport de distances (74) permet de calculer, en tant que second rapport de distances, le rapport de distances dans un cas où la distance d'observation particulière est fixée à la distance d'observation de référence, et
l'unité de correction de la valeur d'indice (78) permet d'obtenir une valeur d'indice de vaisseau sanguin de la distance d'observation de référence en tant que deuxième valeur d'indice de vaisseau sanguin (97a) par correction des valeurs d'indice de vaisseau sanguin variables en fonction de la distance de la première valeur d'indice de vaisseau sanguin (86a) selon le second rapport de distances.

9. Support lisible par ordinateur non transitoire comprenant un programme de commande pour un endoscope (12) dans lequel le programme de commande est installé dans un dispositif de processeur pour l'endoscope (12), le programme amenant le dispositif de processeur à fonctionner comme :
une unité d'acquisition de distance (72) qui permet d'obtenir une première distance d'observation (L1) qui est une distance d'observation non amplifiée entre une partie d'extrémité distale (12d) de l'endoscope (12) et une cible d'observation (82) et au moins une distance d'observation particulière (L2) qui est une distance d'observation amplifiée ;
une unité de calcul de rapport de distances (74) qui permet de calculer un rapport de distances (D) entre la première distance d'observation (L1) et la distance d'observation particulière (L2) ;
une unité d'acquisition de signal d'image (54) qui permet d'obtenir un signal d'image (84) par imagerie de la cible d'observation (82) avec l'endoscope (12) au niveau de la première distance d'observation (L1) ;
une unité de calcul de la valeur d'indice du vaisseau sanguin (76) qui permet de calculer une première valeur d'indice de vaisseau sanguin (86a) d'un vaisseau sanguin de la cible d'observation (82) par numérisation de données relatives à un vaisseau sanguin à partir d'un signal d'image (84) obtenu au niveau de la première distance d'observation (L1) ;
**caractérisé en ce que** :
la première valeur d'indice de vaisseau sanguin (86a) comprend des valeurs d'indice de vaisseau sanguin invariables en fonction de la distance et des valeurs d'indice de vaisseau sanguin variables en fonction de la distance ; et **en ce que**
le programme amène en outre l'ordinateur à fonctionner comme : une unité de correction de la valeur d'indice (78) configurée pour calculer une deuxième valeur d'indice de vaisseau sanguin (97a) au niveau de la distance d'observation particulière (L2) ;
dans lequel l'unité de correction de la valeur d'indice (78) est configurée pour recevoir la première valeur d'indice de vaisseau sanguin (86a) de la distance d'observation non amplifiée (L1) et pour corriger les valeurs d'indice de vaisseau sanguin variables en fonction de la distance de la première valeur d'indice de vaisseau sanguin (86a) selon le rapport de distances et ne pas modifier les valeurs d'indice de vaisseau sanguin invariables en fonction de la distance de la première valeur d'indice de vaisseau sanguin (86a).
